# EUROPÄISCHE PATENTSCHRIFT

(11) **EP 0 439 722 B1**
(45) Veröffentlichungstag und Bekanntmachung des Hinweises auf die Patenterteilung: **21.09.1994**
(21) Anmeldenummer: 90123082.1
(22) Anmeldetag: 03.12.1990
(51) Int. Cl.: C07C 69/76, C07C 67/56

(54) **Verfahren zur Aufarbeitung von rohem Veresterungsgemisch**
Process for the treatment of a crude esterification mixture
Procédé de traitement d'un mélange d'éstérification brut

(30) Priorität: 01.02.1990 DE 4002949
(43) Veröffentlichungstag der Anmeldung: 07.08.1991
(73) Patentinhaber: RWE-DEA Aktiengesellschaft für Mineraloel und Chemie, 22204 Hamburg (DE)
(72) Erfinder: Osterburg, Günther, W-4137 Rheurdt (DE); Reith, Wolfgang, Dr., W-4170 Geldern 3 (DE); Urban, Thomas, W-4250 Bottrop (DE)
(74) Vertreter: Schupfner, Gerhard D.

(56) Entgegenhaltungen:
- DE-A- 2 701 062
- FR-A- 1 469 997

## Beschreibung

Die Erfindung betrifft ein Verfahren zur Aufarbeitung von rohem Veresterungsgemisch, das durch kontinuierliche Veresterung primärer C6- bis C14-Alkohole mit Tricarbonsäuren bzw. deren Anhydriden in Gegenwart von zinn-, titan-, oder zirkon-organischen Verbindungen als Veresterungskatalysatoren erhalten wurde, durch Abdestillation des nicht umgesetzten Alkohols, Abkühlung auf 100 °C und darunter, Abstreifen des Restalkohols aus dem Estergemisch unter Wiederaufheizung, Abkühlung auf unter 80 °C, Zugabe von Aktivkohle, Filtrierung und Trocknung.

Es ist bekannt, Ester, die insbesondere als Weichmacher dienen, durch Veresterung der entsprechenden primären Alkohole mit Di- bzw. Tricarbonsäuren oder deren Anhydriden in Gegenwart saurer Katalysatoren, wie Schwefelsäure, Toluolsulfonsäure oder auf Aktivkohle niedergeschlagener Titan- oder Zirkonhydroxide oder von Titan-, Zirkon- oder Zinnalkoholaten als Veresterungskatalysatoren herzustellen.

Aus der DE-PS 1 273 521 ist ein Verfahren bekannt, wobei nach der im wesentlichen vollständigen Veresterung in Gegenwart von auf Aktivkohle niedergeschlagenen Titan- oder Zirkonhydroxiden zunächst die Hauptmenge an überschüssigem Alkohol abdestilliert, dann der größte Teil des Katalysators vom Ester abgetrennt und schließlich der restliche Alkoholüberschuß in bekannter Weise durch Behandlung mit Wasserdampf vom Ester abgetrennt wird. Hierbei tritt trotz Anwesenheit von restlichem Katalysator bei der Wasserdampfbehandlung keine Verseifung mehr ein.

Aus der DE-AS 23 30 435 ist ein Verfahren bekannt, wonach zur Aufarbeitung von durch Veresterung in Gegenwart von zinn-, titan- oder zirkon-organischen Verbindungen erhaltenen Rohestern neutralisiert, der überschüssige Alkohol abdestilliert, mit Wasserdampf destilliert und getrocknet wird, und die gebildeten festen Bestandteile abfiltriert werden, indem man den vom überschüssigen Alkohol befreiten, 140 bis 250 °C heißen Rohester bei vermindertem Druck gleichzeitig mit wässrigen Lösungen von Alkali- oder Erdalkalihydroxid neutralisiert, durch Versetzen mit Wasser bei vermindertem Druck einer raschen Wasserdampfdestillation unterwirft, bis die Temperatur des Esters auf 80 bis 100 °C abgesunken ist, anschließend den Ester in an sich bekannter Weise trocknet und die gebildeten festen Bestandteile abfiltriert.

Aus der DE-A-2 701 062 ist ein Verfahren bekannt, wonach Veresterungsprodukte von Polycarbonsäuren, z.B. von Mellitsäure, nach Abdestillation der Hauptmenge des nicht umgesetzten Alkohols bei einer Temperatur von 100 bis 340 °C erhitzt und anschließend mit einem Adsorbiermittel, unter anderem Aktivkohle, bei 30 bis 150 °C unter Adsorption mindestens eines Teils der im Rohester enthaltenen Verunreinigungen kontaktiert und der Ester durch fraktionierende Destillation gewonnen wird.

Aus der FR-A-1 469 997 ist ein Verfahren zur Behandlung von Veresterungsgemischen bekannt, wonach Produkte der Veresterung von aliphatischen Alkoholen mit aliphatischen oder aromatischen Carbonsäuren, insbesondere Dicarbonsäuren, in Gegenwart eines Katalysators, u.a. zinn-, titan- oder zirkon-organischen Verbindungen, nach Abdestillieren der Hauptmenge des nicht umgesetzten Alkohols bei vermindertem Druck, mit Aktivkohle bei einer 200 °C nicht übersteigenden Temperatur kontaktiert, die Aktivkohle durch Filtration abgetrennt und das Filtrat fraktioniert wird.

Bei der Veresterung mit einem Katalysator aus Titan-, Zirkon- oder Zinnalkoholaten treten Schwierigkeiten bei der Behandlung mit Wasserdampf auf: es tritt eine katalytisch beschleunigte Verseifung oder Spaltung des Esters ein, die sich in einem Wiederansteigen der Säurezahl bemerkbar machte.

Hierbei führt die Temperaturbelastung der Ester beim Alkoholstrippen in der Stripkolonne bedingt durch die Aktivität des noch anwesenden Veresterungskatalysators zu einer Bildung von Säuregruppen, die - soweit flüchtig - dem Verfahren verloren gehen und sich in dem Stripwasser wieder feststellen lassen, das nunmehr wesentlich saurer ist. Sind die gebildeten Säure- bzw. Anhydridreste nicht flüchtig, steigt die Säurezahl an und führt zu einem weit überhöhten Aktivkohleeinsatz bei der Neutralisation sowie zu langen Filtrationszeiten und damit zu einer erheblichen Einschränkung der Produktionskapazität.

Ausgelöst wird somit der Anstieg der Säurezahl bei der Esteraufarbeitung vorrangig durch Spaltung des Esters bei Temperaturbelastung in Gegenwart von Restkatalysator beim Abstrippen des Restalkohols. Die Temperatur in der Stripkolonne ist aber darauf ausgerichtet, den Partialdruck des freien Alkoholanteils im Weichmacher so zu erhöhen, daß seine weitgehende Abtrennung als Voraussetzung für eine niedere OH-Zahl gewährleistet ist. Eine grundsätzliche Absenkung der Striptemperatur ist somit praktisch nicht möglich. Lediglich bei niedriger siedenden Alkoholanteilen bzw. bei einer weiteren Druckabsenkung in der Stripkolonne kann die Striptemperatur zur Herabsetzung der Bildung von Säuregruppen ohne Nachteile für die OH-Zahl etwas gesenkt werden. Die so erzielte Herabsetzung ist aber nicht ausreichend.

Der Erfindung liegt daher die Aufgabe zugrunde, ein Aufarbeitungsverfahren bereitzustellen, bei dem der Ester nicht oder nur wenig gespalten wird und ein erhöhter Aktivkohleeinsatz sowie lange Filtrierzeiten und damit verbundene Einschränkungen der Produktionskapazität vermieden werden.

Diese Aufgabe wird erfindungsgemäß dadurch gelöst, daß dem Veresterungsgemisch nach Abdestillation des nicht umgesetzten Alkohols und Abkühlung Aktivkohle zugesetzt, in Gegenwart der Aktivkohle der Restalkohol mit Wasserdampf abgestreift und dann der Ester in an sich bekannter Weise abgekühlt, gegebenenfalls mit zusätzlicher Aktivkohle filtriert, und getrocknet wird.

Bevorzugt wird in Gegenwart von 0,1 bis 1 Gew.% Aktivkohle, bezogen auf das Estergemisch, abgestreift.

Überraschenderweise wurde nämlich festgestellt, daß der Veresterungskatalysator durch die Behandlung mit Aktivkohle nicht nur aus dem Ester entfernt, sondern auch in seiner Wirkung inaktiviert wird. Dies war nicht zu erwarten, da bekanntermaßen Titan- und Zirkonhydroxide auf Aktivkohleträger als aktive Veresterungskatalysatoren eingesetzt werden.

Die erfindungsgemäß aufgearbeiteten Veresterungsgemische werden durch kontinuierliche Veresterung primärer C6- bis C14-Alkohole mit Tricarbonsäuren bzw. deren Anhydriden hergestellt. Bei der Alkoholkomponente handelt es sich um C6- bis C14-Alkohole wie n-Hexanol, n-Oktanol und n-Dekanol und insbesondere Gemische dieser Alkohole. Die eingesetzten Säuren sind Tricarbonsäuren, wie die Tribenzolcarbonsäuren. Bevorzugt wird Trimellitsäure eingesetzt.

Die Veresterungstemperatur beträgt in der Regel 180 bis 240 °C. Der nicht umgesetzte Alkohol wird insbesondere bei einer Temperatur von 180 bis 200 °C im Vakuum abdestilliert, sodann auf unter 100 °C, in der Regel auf 60 bis 100 °C abgekühlt, wobei 60 °C die wirtschaftliche Untergrenze darstellt. Anschließend wird insbesondere auf 120 bis 200 °C je nach der Flüchtigkeit der Alkoholkomponente(n) aufgeheizt, erfindungsgemäß in Gegenwart von Aktivkohle der Restalkohol mit Wasserdampf abgestreift und sodann wieder auf unter 80 °C abgekühlt. Hierbei wird eine möglichst tiefe Abkühlung bevorzugt. Die durch Abkühlung eingestellte Temperatur ist jedoch abhängig vom Filtrationsverhalten.

Als Katalysatoren werden titan-, zinn- oder zirkon-organische Verbindungen, insbesondere solche Alkoholate, eingesetzt, wobei die Alkoholkomponente 1 bis 4 Kohlenstoffatome aufweist, wie beispielsweise Zirkontetrabutylat oder Titantetrabutylat.

Die folgenden Beispiele erläutern das erfindungsgemäße Verfahren.

### Vergleichsbeispiel 1 (Stand der Technik)

In einem Rührkessel von 29 m³ Fassungsvermögen wurden 4.540 kg Trimellitsäureanhydrid mit 13.300 kg eines Alkoholgemisches aus gleichen Teilen n-Octanol und n-Decanol (Alkoholüberschuß 30 %) in Gegenwart von 1,5 kg Zirkontetrabutylat als Katalysator in bekannter Weise bei einer Reaktionstemperatur im Rührkessel von 240 °C verestert. Nach 10 Stunden Reaktionszeit wurde die Veresterung mit einer Säurezahl von 0,3 beendet.
Nun wurde der Alkoholüberschuß bei einem sich allmählich auf 5 mbar verbessernden Unterdruck und einer bis auf 220 °C verringerten Temperatur im Rührkessel in 1,5 Stunden abdestilliert und das Estergemisch auf 80 °C abgekühlt. Die Säurezahl betrug danach 0,15. Der Restalkoholgehalt lag bei etwa 2 Gew.%. Das Estergemisch wurde in einen Zwischentank abgelassen und von dort kontinuierlich in einem Vorheizer auf 145 °C erwärmt und anschließend direkt auf den Kopf einer Abstreifkolonne gegeben. Die Abstreifkolonne wurde von unten mit 10 Gew.% Wasserdampf, bezogen auf das Estergemisch, beaufschlagt und bei einem Unterdruck von 90 mbar betrieben. Das am Boden der Abstreifkolonnne abfließende Estergemisch war wasserfrei und hatte die für dieses Estergemisch spezifizierte OH-Zahl. Dagegen war die Säurezahl des Estergemisches von 0,15 auf 0,28 angestiegen.
Nach dem Abkühlen auf 85 °C und einem Zusatz von 10 kg Aktivkohle/t Estergemisch und 0,2 kg Kaliumcarbonat/t Estergemisch wurde nach Filtration eine Säurezahl von 0,19 erhalten. Erst nach einer zweiten Aktivkohlezugabe von 10 kg/t Estergemisch und erneuter Filtration konnte die geforderte Endsäurezahl von 0,1 erreicht werden.

### Beispiel 1 (erfindungsgemäß)

Ein entsprechend dem Vergleichsbeispiel 1 erzeugtes Estergemisch mit einer Säurezahl von 0,15 wurde vor dem Einsatz in die Abstreifkolonne in dem Zwischentank bei 80 °C zunächst mit 2 kg Aktivkohle/t Estergemisch verrührt und anschließend gemeinsam mit der Aktivkohle wie im Beispiel 1 bei 145 °C in der Abstreifkolonne mit Wasserdampf behandelt. Das unten abfließende Estergemisch hatte nur noch einen Anstieg der Säurezahl von 0,15 auf 0,19 erfahren. Nach Zusatz von 10 kg Aktivkohle/t Estergemisch bei 85 °C und nur einer Filtration konnte bereits die geforderte Endsäurezahl von 0,1 erreicht werden. Auf den Zusatz von Kaliumcarbonat als Neutralisationsmittel konnte ganz verzichtet werden, wodurch eine zusätzliche Qualitätsverbesserung des Estergemisches durch Ionenfreiheit erreicht wurde.

### Vergleichsbeispiel 2 (Stand der Technik)

In dem gleichen Rührkessel wie in Beispiel 1 wurden 6.350 kg Trimellitsäureanhydrid mit 13.500 kg eines Alkoholgemisches aus n-Hexanol, n-Octanol und n-Decanol (Alkoholüberschuß 25 %) in Gegenwart von 1,5 kg Zirkontetrabutylat als Katalysator in bekannter Weise bei einer Reaktionstemperatur im Rührkessel von 205 °C verestert. Nach einer Reaktionszeit von insgesamt 17 Stunden wurde die Veresterung mit einer Säurezahl von 0,3 beendet. Nun wurde der Alkoholüberschuß bei gleichbleibender Temperatur im Rührkessel und einem sich allmählich auf 5 mbar verbessernden Unterdruck in einer Stunde abdestilliert und das Estergemisch auf 80 °C abgekühlt. Die Säurezahl betrug danach 0,15. Der Restalkoholgehalt war ähnlich wie im Vergleichsbeispiel 1.
Dieses Estergemisch wurde aus einem Zwischentank kontinuierlich in einem Vorheizer auf 145 °C erwärmt und anschließend direkt auf den Kopf einer Abstreifkolonne gegeben. Die Abstreifkolonne wurde von unten mit 8 Gew.% Wasserdampf bezogen auf das Estergemisch beaufschlagt und bei einem Unterdruck von 90 mbar betrieben.
Das am Boden der Abstreifkolonne abfließende Estergemisch war wasserfrei und hatte die für dieses Estergemisch spezifizierte OH-Zahl. Dagegen war die Säurezahl von 0,15 auf 0,33 angestiegen. Der Einsatz von Neutralisationshilfsmitteln wie z.B. Kaliumcarbonat war bei diesem Estergemisch aus Qualitätsgründen nicht erlaubt.
So wurde nach dem Abkühlen auf 85 °C durch Zusatz von 10 kg Aktivkohle/t Estergemisch und anschließender Filtration die Säurezahl zunächst auf 0,25, nach einer zweiten Zugabe von 10 kg Aktivkohle/t Estergemisch und Filtration auf 0,18 gesenkt und erst nach einer dritten Zugabe von 10 kg Aktivkohle/t Estergemisch mit nochmaliger Filtration die geforderte Endsäurezahl von 0,1 erreicht.

### Beispiel 2 (erfindungsgemäß)

Ein entsprechend dem Vergleichsbeispiel 2 erzeugtes Estergemisch mit einer Säurezahl von 0,15 wurde vor dem Einsatz in die Abstreifkolonne in dem Zwischentank bei 80 °C zunächst mit 2 kg Aktivkohle/t Estergemisch verrührt und anschließend gemeinsam mit Aktivkohle bei 145 °C in der Abstreifkolonne mit Wasserdampf behandelt. Das unten abfließende Estergemisch hatte nur noch einen Säurezahlanstieg von 0,15 auf 0,18 erfahren.
Nach Zusatz von nur 10 kg Aktivkohle/t Estergemisch bei 85 °C und nur einer Filtration konnte bereits die geforderte Endsäurezahl von 0,1 erreicht werden.

## Patentansprüche

1. Verfahren zur Aufarbeitung von rohem Veresterungsgemisch, das durch kontinuierliche Veresterung primärer C6- bis C14-Alkohole mit Tricarbonsäuren bzw. deren Anhydriden in Gegenwart von zinn-, titan- oder zirkonorganischen Verbindungen als Veresterungskatalysatoren erhalten wurde, durch Abdestillation des nichtumgesetzten Alkohols, Abkühlung auf 100 °C und darunter, Abstreifen des Restalkohols aus dem Estergemisch unter Wiederaufheizung, Abkühlen auf unter 80 °C, Zugabe von Aktivkohle, Filtrierung und Trocknung,
**dadurch gekennzeichnet**,
daß man dem Veresterungsgemisch nach Abdestillation der Hauptmenge des nicht umgesetzten Alkohols und Abkühlung Aktivkohle zusetzt, in Gegenwart der Aktivkohle den Restalkohol mit Wasserdampf abstreift, dann in an sich bekannter Weise den Ester abkühlt, gegebenenfalls mit zusätzlicher Aktivkohle filtriert und trocknet.

2. Verfahren nach Anspruch 1,
**dadurch gekennzeichnet**,
daß man in Gegenwart von 0,1 bis 1 Gew.% Aktivkohle, bezogen auf das Estergemisch, abstreift.

3. Verfahren nach Anspruch 1 oder 2
zur Aufarbeitung eines Veresterungsgemischs der Veresterung der angegebenen Alkohole mit Trimellitsäure.

## Claims

1. A process for purifying a crude mixture produced by continuous esterification of primary C₆ to C₁₄ alcohols with tricarboxylic acids or the anhydrides thereof in the presence of organo-tin, -titanium or -zircon compounds as esterification catalysts, which process comprises distilling off the unreacted alcohol, cooling to 100 °C and less, stripping the residual alcohol from the ester mixture while reheating, cooling to less than 80 °C, adding activated carbon, filtering and drying,
characterized by adding activated carbon to the esterification mixture after the major portion of the unreacted alcohol has been distilled off and after cooling, stripping the residual alcohol with steam in the presence of the activated carbon, and, as known per se, cooling the ester, optionally filtering it through an additional amount of activated carbon, and drying.

2. The process of claim 1, wherein stripping is performed in the presence of 0.1 to 1 wt.-% activated carbon, referring to the ester mixture.

3. The process of claim 1 or 2 comprising the purification of a mixture produced by esterification of the alcohols cited hereinabove with trimellitic acid.

## Revendications

1. Procédé de traitement d'un mélange brut qui a été obtenu par estérification continue d'alcools primaires en C₆ à C₁₄ avec des acides tricarboxyliques ou leurs anhydrides en présence de composés organiques d'étain, de titane ou de zirconium comme catalyseurs d'estérification, par élimination par distillation de l'alcool n'ayant pas réagi, refroidissement à 100°C et au-dessous, élimination par entraînement de l'alcool résiduel du mélange d'esters avec reprise du chauffage, refroidissement à moins de 80°C, addition de charbon actif, filtration et séchage,
caractérisé en ce qu'on ajoute du charbon actif au mélange obtenu par estérification après élimination par distillation de la majeure partie de l'alcool n'ayant pas réagi et refroidissement, on chasse l'alcool résiduel par entraînement à la vapeur d'eau en présence de charbon actif puis on refroidit l'ester d'une manière connue, on le filtre éventuellement avec une quantité additionnelle de charbon actif et on le sèche.

2. Procédé suivant la revendication 1, caractérisé en ce qu'on effectue l'entraînement en présence de 0,1 à 1 % en poids de charbon actif par rapport au mélange d'esters.

3. Procédé suivant la revendication 1 ou 2, pour le traitement d'un mélange obtenu par estérification des alcools indiqués avec l'acide trimellitique.
